# EUROPEAN PATENT APPLICATION

(11) **EP 4 831 636 A1**
(43) Date of publication of application: **28.10.2026**
(21) Application number: 25172609.7
(22) Date of filing: 25.04.2025
(51) Int. Cl.: G01N 33/574, G01N 33/68

(54) **IMPROVED IMMUNOPEPTIDOME METHOD TO IDENTIFY NEOEPITOPES FOR CANCER IMMUNOTHERAPY AND NEOEPITOPES AS IDENTIFIED**

(71) Applicant: BioCopy AG, 4057 Basel (CH)
(72) Inventor: Krauss, Jan, 80331 Munich (DE); Klatt, Martin, 12163 Berlin (DE); Zaghla, Badeel, 77933 Lahr (DE)
(74) Representative: Krauss, Jan

(57) **Abstract**

The present invention relates to an improved method for identifying at least one HLA ligand neoantigen peptide derived from at least one mutated antigen protein, as well as neoantigen peptides as identified with the method. Further provided are neoantigen binding proteins, nucleic acid molecules and vectors, recombinant or genetically modified cells, and pharmaceutical compositions or vaccines and their uses in medicine, in particular in the treatment of cancers that express neoantigen peptides or proteins.

## Description

The present invention relates to an improved method for identifying at least one HLA ligand neoantigen peptide derived from at least one mutated antigen protein, as well as neoantigen peptides as identified with the method. Further provided are neoantigen binding proteins, nucleic acid molecules and vectors, recombinant or genetically modified cells, and pharmaceutical compositions or vaccines and their uses in medicine, in particular in the treatment of cancers that express neoantigen peptides or proteins.

### Background of the invention

Immunotherapies such as adoptive T cell therapies or neoantigen/neoepitope vaccinations hold promise for treating cancer by targeting tumor-specific or tumor-associated antigens presented on cancer cells (1-3). These therapies rely on the recognition of peptides processed by the proteasome and presented on HLA molecules (pHLA) (4).

Neoepitopes are a class of major histocompatibility complex (MHC) bound peptides. They represent the antigenic determinants of neoantigens. Neoepitopes are recognized by the immune system as targets for T cells and can elicit immune response to cancer. There is increasing evidence that immune recognition of neoepitopes produced by cancer-specific mutations is a key mechanism for the induction of immune-mediated tumor rejection.

A relatively novel strategy against cancer is epitope selection for mutanome-directed individualized cancer immunotherapy (Vormehr M, Diken M, Boegel S, Kreiter S, Türeci Ö, Sahin U (April 2016). "Mutanome directed cancer immunotherapy". Current Opinion in Immunology. 39: 14-22. doi:10.1016/j.coi.2015.12.001. PMID 26716729). Individualized cancer immunotherapy leverages the adaptive immune system by targeting T cells to tumor cells that have a tumor specific mutant antigen (neoantigen) with neoepitopes recognized by a receptor on T cells, and individualized cancer immunotherapy includes vaccination with tumor mutation-derived neoepitopes.

WO2016040900A1 discloses a vaccine formed using neoantigen peptides and an adjuvant or dendritic cells (DC) autologous to a subject.

WO2019050994A1 discloses peptide sequences of tumor neoantigens that are obtained by sequencing the tumor cells of the subject. The peptide sequences are input into a machine-learned presentation model to generate presentation likelihoods for the tumor neoantigens, each presentation likelihood representing the likelihood that a neoantigen is presented by an MHC allele on the surfaces of the tumor cells of the subject. A subset of the neoantigens is selected based on the presentation likelihoods.

WO2021141374A1 discloses a method and a system for screening for neoantigens, and uses thereof, more specifically to a method and a system which screen for, as a diagnostic and/or treatment target, neoantigens the expressions of which are necessary for cell survival and/or which are derived from the same genes in all cells of the cancer tissue.

Despite their crucial role, pHLA target sequences for individualized neoantigen vaccination studies are often predicted by algorithms rather than experimentally since these vaccines must be produced in a time-efficient manner (3).

However, prediction algorithms although widely established still carry the risk of creating false positive results (5). Similarly, clinical effects following T cell responses like immune evasion by HLA loss of heterozygosity (HLA-LOH) are regularly explored by defining neoepitopes through prediction algorithms (6, 7).

It is therefore an object of the present invention to provide a method to identify neoantigens and to provide these neoantigens that are validated and can reliably be used for a further development of cancer therapy, such as, for example, immunotherapy. Other objects of the present invention will become apparent to the person of skill when studying the specification of the present invention.

In a first aspect thereof, the object of the present invention is solved by providing an improved method for identifying at least one HLA ligand neoantigen peptide derived from at least one mutated antigen protein, comprising the steps of a) co-transfecting a suitable mammalian cell with mRNA from the at least one mutated antigen and mRNA from at least one preselected HLA allele, b) culturing the cell to express HLA/neoantigen peptide complexes, c) isolating the HLA/neoantigen peptide complexes from the culture, d) separating the neoantigen peptide from the HLA proteins using concentrations of between 40 and 50% of acetonitrile, e) analyzing the at least one neoantigen peptide comprising mass spectrometry (MS) analysis, and f) identifying the least one neoantigen peptide.

Preferred is the method according to the present invention, wherein the amount of HLA/neoantigen peptide complex as isolated is higher than about 5 fmol per neoantigen peptide, more preferably, wherein the amount of between about 500,00 and 10⁶ cells is used for the identification after culturing in step b).

Further preferred is the method according to the present invention, wherein the at least one neoantigen peptide as identified comprises a mutation compared to the wildtype sequence of the antigen, and wherein non-mutated peptides of the antigen are not presented in the context of the respective HLA allele. That is, as a wildtype, all these peptides sequence can not bind to the corresponding HLA-type.

In a second aspect thereof, the object of the present invention is solved by providing a neoantigen peptide, comprising an amino acid sequence selected from the group consisting of any one of SEQ ID NO: 1 to SEQ ID NO: 5, wherein the peptide has the ability to bind to an MHC class I molecule and/or wherein said peptide, when bound to said MHC, is capable of being recognized by CD8 T cells, and preferably is selected from the group consisting of SEQ ID NO: 4, and SEQ ID NO: 5, preferably further selected from the group consisting of SEQ ID NO: 7, 8, 9, 15, 17, 29, 31, 32, 33, 37, 39, 44, and 45.

In a third aspect thereof, the object of the present invention is solved by providing a neoantigen binding protein that specifically binds to a neoantigen peptide comprising or consisting of an amino acid sequence according to any one of SEQ ID NO: 1 to SEQ ID NO: 5 and preferably to a sequence selected from the group consisting of SEQ ID NO: 4, and SEQ ID NO: 5, preferably further selected from the group consisting of SEQ ID NO: 7, 8, 9, 15, 17, 29, 31, 32, 33, 37, 39, 44, and 45. when said antigenic peptide is in a complex with a major histocompatibility complex (MHC) protein.

Preferred is the neoantigen binding protein according to the present invention, wherein the antigen binding protein is an antibody or a binding fragment thereof, a bispecific, a trispecific, such as a trispecific bi-paratopic, multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain/nanobody^{®}, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).

The present invention further provides a nucleic acid molecule encoding the neoantigen peptide according to the present invention or the neoantigen binding protein according to the present invention or at least one component thereof. Further provided is a vector comprising a nucleic acid molecule according to the present invention, such as, for example, an expression vector comprising and expressing the nucleic acid molecule.

**In** a fourth aspect thereof, the object of the present invention is solved by providing a recombinant or genetically modified cell comprising and optionally expressing the nucleic acid molecule according to the present invention or the vector according to the present invention, wherein preferably the cell is a T cell, a hybridoma cell, or an antibody excreting cell.

In a fifth aspect thereof, the object of the present invention is solved by providing a method for producing the neoantigen peptide according to the present invention, the neoantigen binding protein according to the present invention or the at least one component thereof, comprising a) culturing a host cell according to the present invention under conditions effective to express the neoantigen peptide or the neoantigen binding protein or component thereof; and b) obtaining the expressed neoantigen peptide or the neoantigen binding protein or component thereof from said host cell or the culture medium of said host cell.

In a sixth aspect thereof, the object of the present invention is solved by providing a method for producing a pharmaceutical composition comprising formulating the neoantigen peptide according to the present invention, the neoantigen binding protein according to the present invention or the at least one component thereof or the recombinant or genetically modified host cell according to the present invention with a suitable pharmaceutically acceptable carrier, or a pharmaceutical composition as produced.

In a seventh aspect thereof, the object of the present invention is solved by providing a pharmaceutical composition, produced according to the present invention, preferably a vaccine comprising at least one neoepitope peptide or protein according to any one of SEQ ID NO: 1 to 5 and 34, and preferably selected from the group consisting of SEQ ID NO: 4, and SEQ ID NO: 5, more preferably further comprising a peptide selected from the group consisting of SEQ ID NO: 7, 8, 9, 15, 17, 29, 31, 32, 33, 37, 39, 44, and 45.or an RNA or DNA molecule encoding the neoepitope peptide or the underlying protein thereof.

Preferred is the pharmaceutical composition or vaccine according to claim 19 wherein the neoepitope peptide has been identified by a method according to the present invention.

In an eighth aspect thereof, the object of the present invention is solved by providing the neoantigen peptide according to the present invention, the neoantigen binding protein according to the present invention, the nucleic acid molecule according to the present invention, the vector according to the present invention, the recombinant or genetically modified cell according to the present invention, or the pharmaceutical composition or vaccine according to the present invention for use in medicine, or for use in the manufacture of a medicine.

Preferred are the neoantigen peptide according to the present invention, the neoantigen binding protein according to the present invention, the nucleic acid molecule according to the present invention, the vector according to the present invention, the recombinant or genetically modified cell according to the present invention, or the pharmaceutical composition or vaccine according to the present invention for use in the prevention or treatment for use the treatment of cancer, wherein the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.

In a nineth aspect thereof, the object of the present invention is solved by providing a kit comprising a) a container comprising a pharmaceutical composition or vaccine according to the present invention in solution or in lyophilized form; and b) optionally, a second container containing a diluent or reconstituting solution for the lyophilized formulation; and c) optionally, at least one more peptide selected from the group consisting of SEQ ID NO: 1 to 5 and 34 and SEQ ID NO: 6 to 33 and 35 to 45.

As mentioned above, in a first aspect thereof, the present invention relates to an improved method for identifying at least one HLA ligand neoantigen peptide derived from at least one mutated antigen protein.

In the context of the present invention, the term "antigen protein" designates the mutated or non-mutated polypeptide that is the origin of the neoantigen as identified. The term "mutated antigen protein" designates the mutated polypeptide that is the origin of the neoantigen as identified. As an example, antigen protein Notch1 defines the non-mutated polypeptide that is the origin of the Notch1 neoantigen (SEQ ID NO: 1) as identified. The term mutated antigen protein defines the antigen protein Notch1 comprising at least the mutation V1575L.

The method according to the invention then comprises a first step of co-transfecting a suitable mammalian cell with mRNA from the at least one mutated antigen and mRNA from at least one preselected HLA allele.

In general, any suitable cell that is capable of being transfected and to produce the HLA/neoantigen peptide complexes may be used. Preferred are mammalian cells, such as human cells. HLA class I molecules are expressed on all nucleated cells and associate with short peptides (8-15 amino acids in length) derived from both self and foreign antigens. Preferred is the method according to the present invention, wherein the cell that expresses the HLA/neoantigen peptide complexes is selected from the group of COS-7, AML14, JMN, Jurkat, MCF-7, and BV173 cells.

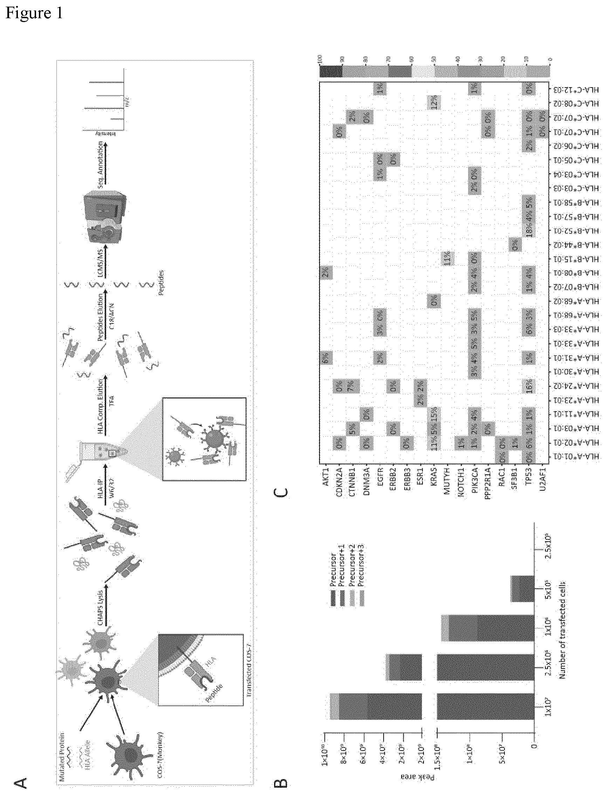

In an embodiment, the inventors first co-transfected monkey fibroblast COS-7 cells with mRNA from both a mutated antigen and one HLA allele using mRNA co-electroporation (Fig. 1A). The inventors then confirmed a sufficient transfection efficiency of at least 90% for all HLA alleles as evaluated. Transfection can be done using any suitable method, such as plasmid transfection using Lipofectamine Reagent (Thermo Fisher Scientific), siportamine (Ambion), ViaFect^{™} Reagent, or FuGENE^{®} (HD) Reagent. Other transfections may be achieved using viruses or co-electroporation, for example mRNA-co-electroporation as above. Approximately 10 × 10⁶ cells can be electroporated per preparation.

In the next step, the cells are cultured to express the recombinant HLA/neoantigen peptide complexes. This can be done in standard media that may be preselected depending on the type of cell that is used. Suitable media are commercially available, and may include RPMI1640 (Gibco), DMEM or MEM (ThermoFisher). The media may be supplemented with antibiotics for selection and/or serum.

In an embodiment, the inventors cultured and maintained co-transfected monkey fibroblast COS-7 cells in RPMI 1640 medium (Gibco) supplemented with penicillin-streptomycin (Pen-Strep, Gibco) and 10% fetal bovine serum (FBS, GeminiBio). The cells were plated in six-well non-tissue culture-treated plates for overnight incubation.

In the following step, the HLA/neoantigen peptide complexes are isolated from the cell culture. This can be done by suitable centrifugation and washing of the cells, followed by a lysis, for example chemical or mechanical, and subsequent collection of supernatants.

In an embodiment, the cells were harvested by incubation with 1 mM EDTA (Millipore Sigma) at 37°C for 5 minutes, followed by pelleting and a single wash in ice-cold RPMI medium (Gibco) and three washes in ice-cold PBS (Gibco). The cells were lysed in 8 ml of 1% CHAPS (Millipore Sigma) for 1 hour at 4°C, after which the lysates were centrifuged at 20,000g for 1 hour at 4°C, and the supernatant was collected.

Then, the complexes are suitably isolated from the supernatant, preferably by using immunopurification comprising MHC I or MHC I-complex-specific antibodies that are coupled or conjugated to a matrix, such as particles or magnetic particles or a matrix of a column, such as sepharose. Based on the antibodies, the complexes are bound to the matrix. In an embodiment, W6/32 antibody (Bio X Cell) was conjugated to 40 mg of CN Br-activated sepharose (Cytiva) and incubated with the protein lysate overnight.

After binding to the matrix, HLA complexes along with binding peptides are then suitably eluted from the matrix and thus isolated from the cell culture. Elution may then be performed using acidic conditions, such as 1% TFA.

In a next step, the neoantigen peptide is separated from the HLA proteins. This can be done using HPLC materials, such as HPLC C18 column materials that are loaded with the complexes, washed, and subsequently eluted using increasing concentrations of acetonitrile of between 15% and 50%. Preferred is a separating the from the HLA proteins using concentrations of between 40 and 60%, preferably 40% and 50% of acetonitrile, as these concentrations provided the highest amounts of peptides.

In an embodiment, peptides and HLA-I complexes were separated using C18 column materials (Sep-Pak C18 1 cc Vac Cartridge, 50 mg sorbent, 37-55 µm particle size, Waters), which were preconditioned with 80% Acetonitrile (ACN) (Millipore Sigma) and equilibrated with three washes of 0.1% TFA. Samples were loaded onto the columns twice, washed twice with 0.1% TFA, and then eluted in 200 µl of sequentially increased ACN concentrations - 15%, 30%, 40%, and 50% and optionally 60% in 0.1% TFA. The four fractions were combined, dried using vacuum centrifugation, and stored at -80°C until further processing.

In another embodiment, solid-phase extractions were used. For this, disks of column material, such as C18 material, can be prepared. The columns are initially washed, e.g. with 100 µl of 80% ACN in 0.1% TFA and subsequently equilibrated three times with 100 µl of 1% TFA. These disks are then placed at the bottom of a suitable pipette tip. Fluids are passed through the column by centrifugation, and the eluates are collected. Dried samples are then resuspended, loaded onto the columns, washed twice, and then run dry. Finally, the samples are eluted with 60 µl of 40% to 60% Acetonitrile/0.1% TFA, and the resulting sample volume is collected, e.g. using further reduction by vacuum centrifugation.

In the last step, the at least one neoantigen peptide is analyzed comprising mass spectrometry (MS) analysis and identified. Samples are preferably analyzed using high-resolution and high-accuracy liquid chromatography-tandem mass spectrometry (LC-MS/MS). Samples are analyzed against a suitable database in order to identify peptides.

Peptide presentation on human leukocyte antigens (HLAs) is essential for initiating T cell responses and all consequences of this presentation including anti-cancer immunity or subsequent immune escape. Nevertheless, many studies rely on prediction tools rather than biological presentation data to study these effects. Thus, the inventors overexpressed 125 combinations of single mutated full-length antigens and one HLA allele to experimentally validate presentation of mutated and non-mutated HLA ligands. Successful presentation was observed only in 22% of predicted cases with strong implications on previously described downstream effects. For example, the association of HLA loss of heterozygosity (HLA-LOH) with predicted neoepitopes was disrupted for 73 of the 125 combinations. Furthermore, when testing 51 sequences used for mRNA neoepitope vaccinations, the inventors observed a strong imbalance between reported T cell responses and the presentation status of these neoepitopes. These data challenge the inventor's interpretation of clinical neoantigen responses and their related effects such as immune evasion

The inventors solved the problem of insufficient representation of neoepitope antigens as identified using higher concentrations of acetonitrile for the separation of MHC ligands and their respective complexes. This increased overall MHC ligand identifications by 2-fold, increased detection of cancer germline antigen-derived peptides by 50%, and resulted in profound variations in isolation efficacy between different MHC alleles correlating with the hydrophobicity of their anchor residues.

Even in these favorable experimental conditions sufficient pHLA presentation was confirmed in only 22% of 176 tested combinations with highest relevance for the interpretation of clinical data on immune evasion, monitoring of neoepitope T cell responses and vaccine design. These data reaffirm that MS validation of potential T cell target is essential to better understand the underlying mechanisms of T cell response and subsequent immune evasion.

Using the above method, the inventors detected the well-characterized KRAS G12V neoantigen in the context of HLA-A*03:01 in as few as 500,000 cells using untargeted MS (Fig. 1B). Using 10 million COS-7 cells for all following experiments, the inventors minimized the risk of false negative results.

Additionally, the inventors confirmed for another well-studied neoantigen:HLA combination (KRAS G12V:HLA-A*02:01) that non-detection was neither due to insufficient transfection of the target antigen nor the HLA allele. Moreover, all transfected HLA alleles presented on average almost 600 endogenous COS7 ligands ensuring successful transfection of the respective HLA allele.

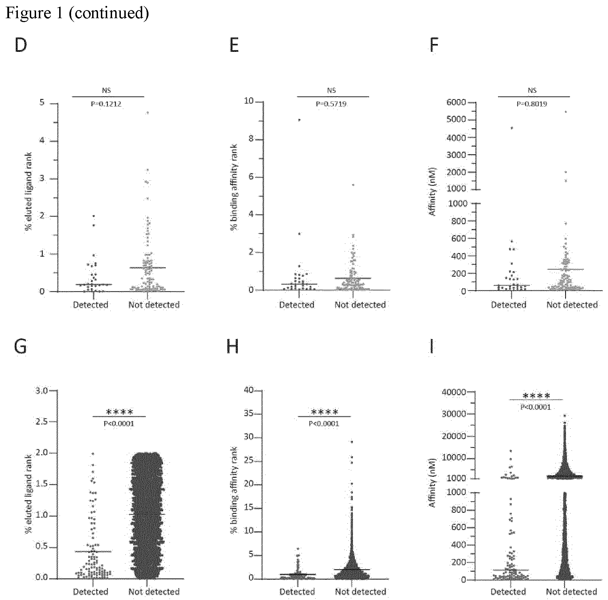

Using the method according to the present invention, the inventors examined 127 neoantigen/HLA combinations for which at least 30 HLA-LOH events were reported in a cohort of 83,644 cancer patients (7). 75% of these combinations were associated with a significant loss of the HLA allele that was predicted to present a public neoepitope from the investigated neoantigen. Of these 127 pairs, 2 combinations (TP53 R175H/HLA-A*02:01 and TP53 Y220C/HLA-A*02:01) were already tested with full length antigens in the COS-7 system (12, 13), leaving 125 untested. Notably, on average only 9.5% of mutated or unmutated HLA ligands predicted to be presented on the transfected HLA allele and derived from the transfected full-length antigens were experimentally validated (Fig. 1C). While some combinations, such as KRAS with HLA-A*02:01, HLA-A*11:01, or HLA-C*08:02, had higher accuracy compared to other alleles, no HLA allele consistently showed better results demonstrating an allele-independent discrepancy between predicted and identified HLA ligands. Although the prediction score had no significant effect on presentation in the mutated peptide group (Fig. 1D-F), non-mutated peptides had a highly significant better chance of getting presented if they achieved better prediction scores (Fig. 1G-H).

In general, the method according to the present invention can be used with any preselected HLA allele of class I. Preferred is the method according to the present invention, wherein the preselected HLA allele is selected from the group of HLA-A*02:01; B*08:01; C*05:01; C*07:01; A*32:01; B*51:01; C*03:04; C*12:03; C*14:02; A*31:01; C*07:02; A*24:02; A*03:01; A*33:03; C*12:03; A*11:01; C*08:02; B*15:01; and A*33:01, in particular A*02:01; B*08:01; C*05:01, C*07:01; B*15:01; and A*33:03.

As mentioned above, preferred is the method according to the present invention, wherein the isolating comprises immunoprecipitation, in particular HLA immunoprecipitation. The isolating according to the present invention preferably comprises the use of C18 column materials, such as C18 cartridges, i.e. materials for use in HPLC. HPLC may also be used after the required technical adjustments.

It was surprisingly found that in the method according to the present invention the amount of HLA/neoantigen peptide complex as isolated is higher than about 5 fmol per neoantigen peptide, preferably higher than about 10 fmol per neoantigen peptide.

It was also surprisingly found that in the method according to the present invention, that the at least one neoantigen peptide as identified comprises a mutation compared to the wildtype sequence of the antigen, and wherein non-mutated peptides of the antigen are not presented in the context of the respective HLA allele. Thus, the present method preferably allows to particularly identify neoantigen peptides that are presented that are mutated when compared to their wildtype sequence. At the same time, these (mutated ones) are the only peptides of the antigen that are presented in the context of the of the respective HLA allele, that is, there is a selective identification of single antigen derived mutated neoantigen peptides. Obviously, these neoantigen peptides are preferred, as the antigen response is ultimately linked with the mutation in the peptide as presented. As a wildtype, all these peptides sequence can not bind to the corresponding HLA-type. These peptides are herein also referred to as "mutation only" neoantigen peptides. Preferably, the mutated amino acid is in an anchor position of the (mutation only) neoantigen peptide.

Therefore, preferred is at least one "mutation only" neoantigen peptide as identified selected from the group consisting of SEQ ID NO: 4, 5, 7, 8, 9, 15, 17, 29, 31, 32, 33, 37, 39, 44, and 45.

It was also surprisingly found that in the method according to the present invention the amount of between about 500,00 and 10⁶ cells as used is sufficient for the identification after culturing in step b).

Yet another aspect of the present invention relates to a neoantigen peptide, comprising an amino acid sequence selected from the group consisting of any one of SEQ ID NO: 1 to SEQ ID NO: 5, wherein the peptide has the ability to bind to an MHC class I molecule and/or wherein said peptide, when bound to said MHC, is capable of being recognized by CD8 T cells. Preferably, the peptide has an overall length of from 8 to 20 amino acids.

Yet another aspect of the present invention relates to a neoantigen peptide, comprising an amino acid sequence selected from the group consisting of any one of SEQ ID NO: 1 to SEQ ID NO: 45, wherein the peptide has the ability to bind to an MHC class I molecule and/or wherein said peptide, when bound to said MHC, is capable of being recognized by CD8 T cells. Preferably, the peptide has an overall length of from 8 to 20 amino acids.

Preferred is a neoantigen peptide according to the present invention that is selected from the group consisting of SEQ ID NO: 4, and SEQ ID NO: 5, and preferably further selected from the group consisting of SEQ ID NO: 7, 8, 9, 15, 17, 29, 31, 32, 33, 37, 39, 44, and 45.

In the context of the present invention, the terms "neoantigens" or " neoepitopes" are used interchangeably, and relate to antigenic peptides showing neoepitopes as described herein.

Notch1, a member of the Notch family, is implicated in many types of cancer, including breast cancer (especially triple-negative breast cancer), leukemias, brain tumors, and many others. Notch1 is tightly connected to many signaling pathways that are therapeutically involved in tumorigenesis. Targeting Notch1 in cancers that harbor high expression levels of Notch1 offers an addition to therapeutic strategies recruited for managing cancer (Gharaibeh, L. et al. Notch1 in Cancer Therapy: Possible Clinical Implications and Challenges. Molecular Pharmacology, Volume 98, Issue 5, 559 - 576).

Mutations in MutY homolog (*MUTYH*) are best known for *MUTYH* associated polyposis and colorectal cancer, but the gene also plays a role in the development of ovarian cancer (Hutchcraft ML, Gallion HH, Kolesar JM. MUTYH as an Emerging Predictive Biomarker in Ovarian Cancer. Diagnostics (Basel). 2021 Jan 6;11(1):84. doi: 10.3390/diagnostics11010084. PMID: 33419231; PMCID: PMC7825630).

TP53, a crucial tumor suppressor gene, is the most commonly mutated gene in human cancers. Aside from losing its tumor suppressor function, mutant p53 (mutp53) often acquires inherent, novel oncogenic functions, which is termed "gain-of-function". Emerging evidence suggests that mutp53 is highly associated with advanced malignancies and poor prognosis, which makes it a target for development of novel cancer therapies. Mutp53 has been found to have immunogenicity and can act as a neoantigen to trigger an immune response (Malekzadeh P, Pasetto A, Robbins PF, Parkhurst MR, Paria BC, Jia L, et al. Neoantigen screening identifies broad TP53 mutant immunogenicity in patients with epithelial cancers. J Clin Investig. 2019;129:1109-14). In ovarian cancer and metastatic colorectal cancer, there are specific T cells against the mutant neoantigen in tumor infiltrating lymphocytes (Deniger DC, Pasetto A, Robbins PF, Gartner JJ, Prickett TD, Paria BC, et al. T-cell Responses to TP53 "Hotspot" Mutations and Unique Neoantigens Expressed by Human Ovarian Cancers. Clin Cancer Res. 2018;24:5562-73).

The *AKT1*^{E17K}-mutant plays an important role in specific ER-positive breast cancers (Smyth LM, et al.; AACR Project GENIE Consortium. Characteristics and Outcome of AKT1E17K-Mutant Breast Cancer Defined through AACR Project GENIE, a Clinicogenomic Registry. Cancer Discov. 2020 Apr;10(4):526-535. doi: 10.1158/2159-8290.CD-19-1209. Epub 2020 Jan 10. PMID: 31924700; PMCID: PMC7125034.). Capivasertib, an oral AKT inhibitor, as monotherapy or combined with fulvestrant is under clinical investigation (phase I study NCT01226316).

Mutations in *CTNNBl,* the gene encoding β-catenin, are common in colon and liver cancers, the most frequent mutation affecting Ser-45 in β-catenin. Puig-Saus, C., et al. (in: Neoantigen-targeted CD8+ T cell responses with PD-1 blockade therapy. Nature 615, 697-704 (2023). https://doi.org/10.1038/s41586-023-05787-1) generated personalized libraries of neoantigen-HLA capture reagents to single-cell isolate the T cells and clone their T cell receptors (neoTCRs). Multiple T cells with different neoTCR sequences (T cell clonotypes) recognized a limited number of mutations in samples from seven patients with long-lasting clinical responses. These neoTCR clonotypes were recurrently detected over time in the blood and tumour.

L. Wang, et al. (in: P4.11D.05 EGFR Neoantigen Peptide Vaccine Combined with Tislelizumab and Chemotherapy for Advanced NSCLC Resistant to EGFR-TKI Therapy, Journal of Thoracic Oncology, Volume 19, Issue 10, Supplement, 2024, Page S391, ISSN 1556-0864, https://doi.org/10.1016/j.jtho.2024.09.707) disclose that neo-antigens produced by EGFR mutations have strong immunogenicity and are ideal antigens for vaccine. The combination of ICI and neo-antigen vaccine is expected to have a synergistic effect which can improve the immune suppression microenvironment and further enhance the anti-tumor immune response. This study aimed to explore the efficacy and safety of neo-antigen vaccine with tislelizumab (TIS) and chemotherapy in the treatment of advanced NSCLC resistant to previous EGFR-TKI treatment (NCT06095934).

Dailey GP, et al. (in: Vaccines targeting ESR1 activating mutations elicit anti-tumor immune responses and suppress estrogen signaling in therapy resistant ER+ breast cancer. Hum Vaccin Immunother. 2024 Dec 31;20(1):2309693. doi: 10.1080/21645515.2024.2309693. Epub 2024 Feb 8. PMID: 38330990; PMCID: PMC10857653) disclose the use of HPLC to demonstrate the presentation of *ESR1* and *ESR1*mut peptides on human ER+ BC cell MHC complexes.

Lu, D., et al. (in: KRAS G12V neoantigen specific T cell receptor for adoptive T cell therapy against tumors. Nat Commun 14, 6389 (2023). https://doi.org/10.1038/s41467-023-42010-1) disclose two TCRs specific for the 9-mer KRAS-G12V mutant neoantigen in the context of HLA-A*11:01. The TCR-T cells are constructed and display cytokine secretion and cytotoxicity upon co-culturing with varied tumor cells expressing the KRAS-G12V mutation. Moreover, 1-2C TCR-T cells show anti-tumor activity in preclinical models in female mice. The 9-mer KRAS-G12V mutant peptide exhibits a distinct conformation from the 9-mer wildtype peptide and its 10-mer counterparts.

Janku F, et al. (in: PIK3CA mutation H1047R is associated with response to PI3K/AKT/mTOR signaling pathway inhibitors in early-phase clinical trials. Cancer Res. 2013 Jan 1;73(1):276-84. doi: 10.1158/0008-5472.CAN-12-1726. Epub 2012 Oct 12. PMID: 23066039; PMCID: PMC3537862) analyze the interaction between PIK3CA mutation H1047R versus other aberrations and response to PI3K/AKT/mTOR axis inhibitors in diverse cancers.

The E41K mutation activates Bruton's tyrosine kinase (BTK). Constitutive activation of BTK is often linked to several forms of B-cell leukemias and lymphomas like chronic lymphocytic leukemia, and diffuse large B cell lymphoma (ABC-DLBCL), suggesting a critical regulatory function of BTK in B-cell development and proliferation. Thus, BTK has been an important pharmacological target for the treatment of various B-cell malignancies.

Serine protease PRSS23 is a protein that has been associated with tumor progression in various types of cancers. Interestingly, PRSS23 is coexpressed with estrogen receptor α (ERα), which is a prominent biomarker and therapeutic target for human breast cancer. Qin et al. (in: Serine protease PRSS23 drives gastric cancer by enhancing tumor associated macrophage infiltration via FGF2. Front Immunol. 2022 Sep 15;13:955841. doi: 10.3389/fimmu.2022.955841. PMID: 36189305; PMCID: PMC9520605) analyzed PRSS23 in two-independent gastric cancer (GC) cohorts.

Radil is an important player for regulating Ras signaling, cell adhesion, and the epithelial mesenchymal transition and may provide new directions for Ras-related anticancer drug development (Byeong Hyeok Choi, et al. Identification of Radil as a Ras binding partner and putative activator, Journal of Biological Chemistry, Volume 296, 2021, 100314, ISSN 0021-9258, https://doi.org/10.1016/j.jbc.2021.100314.).

SDK1 is a prognostic marker in kidney renal clear cell carcinoma, and lung adenocarcinoma
Fu Y, et al. (in: SYT7 acts as an oncogene and a potential therapeutic target and was regulated by ΔNp63α in HNSCC. Cancer Cell Int. 2021 Dec 20;21(1):696. doi: 10.1186/s12935-021-02394-w. PMID: 34930262; PMCID: PMC8691088) disclose the SYT7/ΔNp63α axis as a potential therapeutic target for clinical treatment of HNSCC.

Fu, Z., et al. (in: SYT8 promotes pancreatic cancer progression via the TNNI2/ERRα/SIRT1 signaling pathway. Cell Death Discov. 7, 390 (2021). https://doi.org/10.1038/s41420-021-00779-4) disclose that SYT8 levels are upregulated in patient pancreatic tumors and contributed towards increased cell proliferation, migration, and invasion in vitro and in vivo.

TAS2R43 (Taste 2 Receptor Member 43) is a Protein Coding gene. Diseases associated with TAS2R43 include Inflammatory Bowel Disease and Submandibular Gland Cancer.

Ubiquitin conjugating enzyme E2S (UBE2S), a member of the ubiquitin-conjugating enzyme family, is known to play a pivotal role in tumorigenesis and progression in some tumor types (Bao H, et al. A Pan-Cancer Analysis of UBE2S in Tumorigenesis, Prognosis, Pathway, Immune Infiltration and Evasion, and Therapy Response from an Immune-Oncology Perspective. J Oncol. 2022 May 7;2022:3982539. doi: 10.1155/2022/3982539. PMID: 35578600; PMCID: PMC9107357).

The present invention further relates to the neoantigen peptide according to the present invention, wherein said peptide is fused to a different peptide or protein. Preferably, the peptide is part of a fusion protein, in particular fused to the N-terminal amino acids of the HLA-DR antigen-associated invariant chain (Ii), or fused to (or into the sequence of) an antibody, such as, for example, an antibody that is specific for dendritic cells.

The present invention further relates to the peptides according to the present invention, wherein said peptide is modified, such as glycosylated, and/or includes non-peptide bonds.

Another embodiment of the present invention relates to a non-naturally occurring peptide wherein said peptide consists or consists essentially of an amino acid sequence according to SEQ ID No: 1 to SEQ ID No: 45, preferably consisting or consisting essentially of an amino acid sequence according to SEQ ID No: 1 to SEQ ID No: 5, and has been synthetically produced (e.g. synthesized) as a pharmaceutically acceptable salt. Methods to synthetically produce peptides are well known in the art. The salts of the peptides according to the present invention differ substantially from the peptides in their state(s) in vivo, as the peptides as generated in vivo are no salts. The non-natural salt form of the peptide mediates the solubility of the peptide, in particular in the context of pharmaceutical compositions comprising the peptides, e.g. the peptide vaccines as disclosed herein. A sufficient and at least substantial solubility of the peptide(s) is required in order to efficiently provide the peptides to the subject to be treated. Preferably, the salts are pharmaceutically acceptable salts of the peptides.

Generally, peptides may be synthesized by the Fmoc-polyamide mode of solid-phase peptide synthesis as disclosed by Lukas et al. (Lukas et al., Solid-phase peptide synthesis under continuous-flow conditions, PNAS 78, 5, pp 2791-5, 1981) and by references as cited therein.

Another embodiment of the present invention relates to a neoantigen binding protein that specifically binds to a neoantigen peptide comprising or consisting of an amino acid sequence according to any one of SEQ ID NO: 1 to SEQ ID NO: 45, preferably according to SEQ ID No: 1 to SEQ ID No: 5, and SEQ ID NO: 34, when said antigenic peptide is in a complex with a major histocompatibility complex (MHC) protein.

Preferred is the neoantigen binding protein according to the present invention, wherein the antigen binding protein is an antibody or a binding fragment thereof, a bispecific, a trispecific, such as a trispecific bi-paratopic, multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain/nanobody^{®}, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).

The present invention further relates to neoantigen binding proteins that are specific against the peptides according to the present invention or complexes of said peptides according to the present invention with MHC, and methods of making these.

Optionally, the neoantigen binding proteins carries a further effector function such as an immune stimulating domain or toxin.

Preferred is the neoantigen binding protein according to the present invention, wherein the antigen binding protein is at least bispecific and furthermore binds to an antigen selected from the group consisting of CD3, CD16, NKG2D, CD28, NKp44, NKp46, OX40, GITR, CD137, CD27, CD40L, HVEM or human glucocorticoid-induced TNFR-related protein (GITR).

The present invention further relates to a nucleic acid molecule encoding the neoantigen peptide according to the present invention or the neoantigen binding protein according to the present invention or at least one component thereof. The present invention further relates to the nucleic acid according to the present invention that is DNA, cDNA, RNA or combinations thereof.

The present invention further relates to a vector comprising a nucleic acid molecule according to the present invention or an expression vector capable of expressing and/or expressing a nucleic acid according to the present invention.

The present invention further relates to a recombinant or genetically modified cell, such as a host cell comprising a nucleic acid according to the present invention or a vector or an expression vector as described before. The present invention further relates to the host cell according to the present invention that is an antigen presenting cell, and preferably is a dendritic cell, a T cell, a hybridoma cell, or an antibody excreting cell.

Provided is furthermore a method for producing the neoantigen peptide or the neoantigen binding protein or the at least one component thereof as disclosed herein. The method includes culturing a host cell such as a recombinant host cell that expresses or contains the peptide binding protein or the at least one component thereof. The method furthermore includes isolating or otherwise obtaining the peptide, from the host cell and/or from the culture medium that contains the host cell.

In an aspect, a cDNA encoding a neoantigen peptide or a neoantigen binding protein or the at least one component thereof as disclosed herein as disclosed herein, such as encoding a peptide according to any one of SEQ ID NO: 1 to SEQ ID NO: 45, can be expressed in prokaryotic cells (e.g., bacteria) or eukaryotic cells (e.g., yeast, insect, or mammalian cells), after which the recombinant peptide can be purified.

The present invention further relates to a method for producing a pharmaceutical composition, comprising formulating the neoantigen peptide according to the present inventio, the neoantigen binding protein according to the present invention or the at least one component thereof or the recombinant or genetically modified host cell according to the present invention with a suitable pharmaceutically or therapeutically acceptable excipient or carrier, and a pharmaceutical composition as produced.

Provided herein is a pharmaceutical composition, produced according to the present invention, preferably a vaccine comprising at least one neoepitope peptide or protein according to any one of SEQ ID NO: 1 to 5, and 34 or an RNA or DNA molecule encoding the neoepitope peptide or protein.

Provided herein is a combination product, comprising one or more of the neoantigen peptide or salt thereof, the neoantigen binding protein, the nucleic acid, the expression vector or the recombinant host cell as provided herein.

Particularly preferred is the vaccine according to the present invention, comprising at least 2, preferably 3, 4, 5, 6, 7, 8, 9 or 10 or more antigenic peptides comprising at least one neoepitope peptide or protein according to any one of SEQ ID NOs: 1 to 5 and 34, and optionally at least one of a neoepitope peptide or protein according to any one of SEQ ID NOs: 6 to 33 and 35 to 45.

Further preferred is the vaccine according to the present invention, which is in the form of a dendritic cell vaccine, wherein the dendritic cell is pulsed or loaded with the at least one neoepitope peptide or genetically modified to express at least one neoepitope peptide or neoepitope protein.

The pharmaceutical composition according to the present invention may comprise a suitable adjuvant. The adjuvant may be selected from the group consisting of bacterial DNA-based adjuvants, oil/surfactant based adjuvants, viral dsRNA-based adjuvants and imidazochinilines, and/or a Montanide ISA adjuvant. Further preferred is the pharmaceutical composition for use according to the present invention, wherein the vaccine composition comprises antigen-presenting cells comprising the immunogenically active peptide fragment or a nucleic acid encoding said immunogenically active peptide fragment, as described herein, wherein preferably the antigen-presenting cell is a dendritic cell.

In one aspect, the adjuvant of the pharmaceutical composition or of the combination product provided herein, comprises IFN-alpha, IFN-beta or an interleukin, wherein the interleukin preferably is IL-1 , IL-2, IL-4, IL-7, IL-9, IL-10, IL-12, IL-13, IL-15, IL-17, IL-18, IL-21 , IL-23 or a combination thereof. In another aspect, the adjuvant of the pharmaceutical composition or of the combination product provided herein, is IL-2, IL-12, IL-15, IL-18, IL-21 or a combination thereof.

The term "pharmaceutically or therapeutically acceptable excipient or carrier" refers to a solid or liquid filler, diluent or encapsulating substance which does not interfere with the effectiveness or the biological activity of the active ingredients and which is not substantially toxic to the host, which may be either humans or animals, to which it is administered. Depending upon the particular route of administration, a variety of pharmaceutically acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention. Administration of the composition as a medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra-atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like. **In** the context of the present invention, preferred is an anti-cancer or anti-senescent vaccine as mentioned above.

**In** addition to the aforementioned compounds of the invention, the pharmaceutical composition can contain two or more compounds according to the present invention and also other therapeutically active substances.

The dosage of the pharmaceutical composition according to the present invention can be appropriately selected according to the route of administration, the subject to be administered, the target disease and its severity, age, sex weight, individual differences and disease state. Dosage may be repeated several times a day.

Particularly preferred is the pharmaceutical composition or vaccine according to the present invention, wherein the neoepitope peptide has been identified by a method according to the present invention.

Another aspect of the neoantigen peptide according to the present invention, the neoantigen binding protein according to the present invention, the nucleic acid molecule according to the present invention, the vector according to the present invention, the recombinant or genetically modified cell according to the present invention, or the pharmaceutical composition or vaccine according to the present invention is their use in medicine, in particular for use in the manufacture of a medicament.

Preferred is the use of the neoantigen peptide according to the present invention, the neoantigen binding protein according to the present invention, the nucleic acid molecule according to the present invention, the vector according to the present invention, the recombinant or genetically modified cell according to the present invention, or the pharmaceutical composition or vaccine according to the present invention in the prevention, treatment and/or diagnosis of cancer, wherein the cancer preferably is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.

Preferred is the pharmaceutical composition for use according to the present invention, wherein said prevention or treatment comprises inducing a major histocompatibility complex (MHC) class I-dependent T-cell response.

Another aspect of the present invention relates to a therapeutic or non-therapeutic method for eliciting a CD8-positive T-cell response in a subject, comprising administering to said subject the pharmaceutical composition for use according to the present invention or the pharmaceutical composition according to the present invention.

Another aspect of the present invention relates to a method of preventing or treating a patient against cancer and/or a senescent cell, the method comprising administering to the patient an effective amount of the neoantigen peptide according to the present invention, the neoantigen binding protein according to the present invention, the nucleic acid molecule according to the present invention, the vector according to the present invention, the recombinant or genetically modified cell according to the present invention, and/or the pharmaceutical composition or vaccine according to the present invention.

Preferred is the method according to the present invention, wherein the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.

Preferred is the method according to the present invention, wherein said prevention or treatment comprises inducing a major histocompatibility complex (MHC)-I-dependent T-cell response. Further preferred is the method according to the present invention, wherein the cancer and/or senescent cell is from a tumor presenting neoantigen peptide according to the present invention or expressing the underlying mutated protein thereof, e.g. ER2+ breast cancer.

Preferred is the method according to the present invention, wherein the method is combined with a further cancer treatment, wherein preferably the further treatment is selected from the group consisting of chemotherapy, radiotherapy, treatment with immunostimulating substances, gene therapy, treatment with antibodies and treatment using dendritic cells.

Another aspect of the present invention relates to a kit comprising a) a container comprising a pharmaceutical composition or vaccine according to the present invention in solution or in lyophilized form; and b) optionally, a second container containing a diluent or reconstituting solution for the lyophilized formulation; and c) optionally, at least one more peptide selected from the group consisting of SEQ ID NO: 1 to 5 and 34 and SEQ ID NO: 6 to 33 and 35 to 45.

Yet another aspect of the present invention relates to a kit comprising; a) the pharmaceutical composition for use according to the present invention or the pharmaceutical composition according to the present invention, and b) a second container containing a diluent or reconstituting solution for the lyophilized formulation; and c) optionally, at least one more peptide selected from the group consisting of SEQ ID NO: 1 to 5 and 34 and SEQ ID NO: 6 to 33 and 35 to 45.

Preferred is the kit according to the present invention, further comprising d) a composition comprising at least one second pharmaceutically active ingredient, wherein preferably the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine, wherein preferably the provided compositions are to be administered simultaneously or sequentially.

Preferred is the kit according to the present invention, where the provided compositions are to be administered simultaneously or sequentially.

In the context of the present invention, the inventors optimized co-transfection experiments of one neoantigen and one HLA followed by pHLA isolation and mass spectrometry (MS) analysis to minimize the risk of false positive results to define high confidence neoepitopes (8, 9). These data reaffirm that MS validation of potential T cell target is essential to better understand the underlying mechanisms of T cell response and subsequent immune evasion.

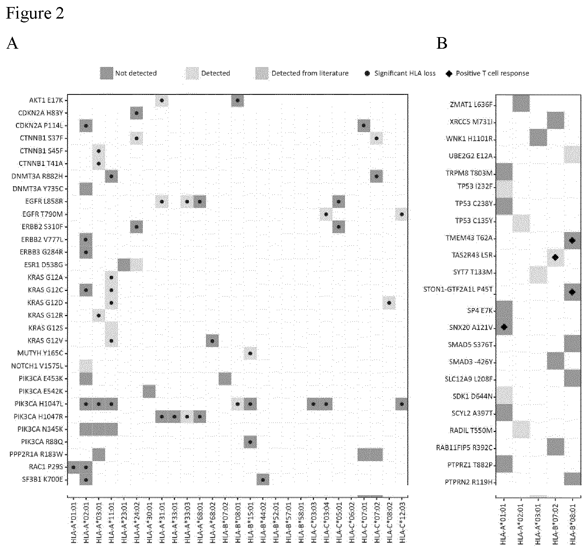

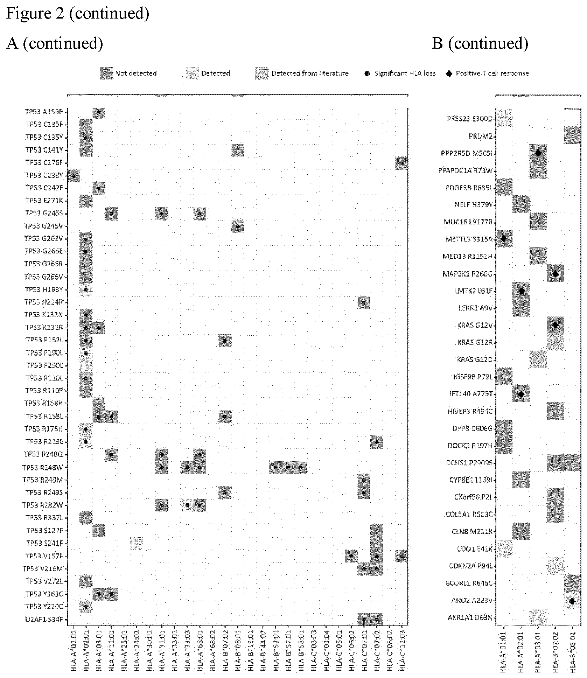

In consequence, only 23.1% of the 125 combinations resulted in successful presentation of a neoepitope independently if HLA-LOH was significantly associated with these pairs or not (Fig. 2A). In retrospect, these data match previous reports that confirmed neoepitope presentation for 23.6% of neoantigen:HLA pairs after the inventors filtered these datasets for combinations that were also predicted to present neoepitopes associated with HLA-LOH (14, 15).

Still, nine previously published KRAS neoepitopes from seven combinations (G12D in HLA-A*11:01, G12D in HLA-C*08:02 as well as G12V, G12C, G12A, G12S, in HLA-A*11:01, and G12R in HLA-A*03:01) (14, 15), were successfully validated. Additionally, 24 undescribed public neoepitopes across 11 HLA alleles from 8 different neoantigens were identified including public neoantigens evolving from resistance associated mutations (ESR1 D538G and T790M EGFR1) (Fig. 2A).

Overall, these data highlight the very limited capacity of prediction algorithms to define public neoepitopes on a larger scale. Thus, downstream analyses associated with predictions like immune evasion through HLA-LOH carry a large potential of generating false positive results.

Similarly, private neoepitopes are regularly used in clinical and preclinical studies for the generation of personalized neoantigen vaccines (3, 16, 17). As these vaccines need to be designed fast, they rely heavily on prediction algorithms and presentation of these private neoepitopes has never been evaluated systematically. Thus, the inventors expanded the inventor's co-transfection experiments to 15-mer minigenes that had been used as mRNA vaccines in a recent phase-I clinical trial which investigated the effect of an adjuvant neoantigen vaccine in combination with checkpoint blockade and standard chemotherapy in pancreatic duct adenocarcinoma (3). The inventors selected mutations that were predicted to be presented on HLA-A*01:01, HLA-A*02:01, HLA-A*03:01, HLA-B*07:02, and HLA-B*08:01 to improve comparability and which included minigenes from 14/16 patients analyzed in this study to avoid any patient-specific bias. This selection yielded 53 combinations for which clinical T cell monitoring data were available that the inventors wanted to correlate with the presentation status of the investigated neoantigens. Of these 53 pairs, 2 combinations (KRAS G12D/HLA-A*03:01 and KRAS G12R/HLA-B*07:02) were already validated by other immunopeptidome studies, leaving 51 untested.

The inventors identified 12 private neoepitopes across five HLA alleles from 51 combinations which resembled perfectly the rate of successful presentations (23.5%) from the inventor's previous experiments (Fig. 2B). Interestingly, even if the sample size was limited, presentation of private neopeptides was significantly associated with better binding prediction scores. Surprisingly, the inventor's analysis found no significant correlation between neoepitope presentation and reported T cell responses. Only two of the 12 detected neoepitopes corresponded with positive immune responses reported in clinical trials, while the remaining 10 did not. In other words, in the inventor's selection of 51 combinations the positive rate for T cell responses was 16.7% (2/12) in the group of presented neoepitopes and 23% (9/39) in the group of non-presented neoepitopes.

For example, neoepitopes derived from the combination of B*07:02:KRAS G12V against which a positive T cell response was reported, were neither detected in the inventor's study nor in two previous immunopeptidome studies that explored public neoepitopes derived from this combination (13, 14).

Additionally, polyclonal T cell responses were reported and presumably mutation-specific T cell receptors isolated against the PLXNB3 Q1897K minigene in the context of HLA-A*32:01.

Nevertheless, the inventor's pipeline did not detect any HLA-A*32-restricted HLA ligands derived from this minigene, whereas a peptide from the CMV pp65 antigen was presented on this allele as a positive control.

Here, the inventors demonstrate a significant discrepancy between predicted and experimentally validated neoepitopes as well as their reported recognition by T cells. Using full-length neoantigens and single HLA alleles the inventors minimized biases through antigen and HLA allele competition, processing and expression, if multiple minigenes are combined in single vectors (14). Only 22% of combinations resulted in successful presentation of the predicted neoepitopes which nevertheless led to the definition of 24 previously undescribed public neoepitopes and confirmed nine previously reported public neoepitopes (14,15). In contrast, 78% of public neoepitopes that were predicted and potentially associated with HLA-LOH were not presented and thus not responsible for the observed HLA-LOH.

As many studies on neoantigen target selection and biomarker development for immune checkpoint blockade rely heavily on prediction algorithms (18, 19) the inventor's results call for better integration of immunopeptidome data as neoepitope predictions might not provide a solid foundation for biological interpretation of downstream effects of neoantigen presentation. Furthermore, T-cell in vitro assays can lead to positive results independent of the actual target presentation if peptide pulsing or tandem minigenes are used which both do not resemble processing and presentation of full-length antigens (20).

Finally, the inventors examined the correlation of neoantigen presentation with clinical T cell response assessment in a neoantigen vaccination study in pancreatic cancer. The inventors found no significant correlation between neoepitope presentation and reported T cell responses including many examples of observed T cell responses without sufficient presentation of the suggested epitope. This included a supposedly HLA-B*07 restricted epitope from the KRAS G12V antigen which in three independent studies has been demonstrated not to be processed and presented highlighting the risk of false positive responses deducted from in vitro T cell assays. Even for an HLA-A*32 restricted target against which several presumably mutation-specific TCRs were isolated, the suggested minigene did not generate any neoepitopes in the context of HLA-A*32:01.

In summary, the inventor's data highlight the need to critically test prediction results for neoepitopes as conclusions drawn from these datasets contain a large fraction of false positive results. This challenges many existing neoantigen datasets and has significant implications not only for cancer immunology but also for broader cell biology fields, including autoimmunity and infectious diseases, where prediction tools are widely used. In contrast, positive data such as the 24 newly identified public neoepitopes form a strong basis for developing new treatment strategies including immunotherapies against resistance-associated mutations and suggest that personalized vaccination strategies will also benefit from the systematic evaluation of target presentation during the selection process.

The present invention relates to the following items:
Item 1. An improved method for identifying at least one HLA ligand neoantigen peptide derived from at least one mutated antigen protein, comprising the steps of
   a) co-transfecting a suitable mammalian cell with mRNA from the at least one mutated antigen and mRNA from at least one preselected HLA allele,
   b) culturing the cell to express HLA/neoantigen peptide complexes,
   c) isolating the HLA/neoantigen peptide complexes from the culture,
   d) separating the neoantigen peptide from the HLA proteins using concentrations of between 40 and 50% of acetonitrile,
   e) analyzing the at least one neoantigen peptide comprising mass spectrometry (MS) analysis, and
   f) identifying the least one neoantigen peptide.
Item 2. The method according to Item 1, wherein the cell that expresses the HLA/neoantigen peptide complexes is selected from the group of COS-7, AML14, JMN, MCF-7, and BV173 cells.
Item 3. The method according to Item 1 or 2, wherein the preselected HLA allele is selected from the group of HLA-A*02:01; B*08:01; C*05:01; C*07:01; A*32:01; B*51:01; C*03:04; C*12:03; C*14:02; A*31:01; C*07:02; A*24:02; A*03:01; A*33:03; C*12:03; A*11:01; C*08:02; B*15:01; and A*33:01, in particular A*02:01; B*08:01; C*05:01, C*07:01; B*15:01; and A*33:03.
Item 4. The method according to any one of Items 1 to 3, wherein the isolating comprises immunoprecipitation, in particular HLA immunoprecipitation.
Item 5. The method according to any one of Items 1 to 4, wherein the isolating comprises the use of C18 HPLC column material.
Item 6. The method according to any one of Items 1 to 5, wherein the amount of HLA/neoantigen peptide complex as isolated is higher than about 5 fmol per neoantigen peptide.
Item 7. The method according to any one of Items 1 to 6, wherein the at least one neoantigen peptide as identified comprises a mutation compared to the wildtype sequence of the antigen, and wherein non-mutated peptides of the antigen are not presented in the context of the respective HLA allele.
Item 8. The method according to any one of Items 1 to 7, wherein the amount of between about 500,00 and 10⁶ cells is used for the identification after culturing in step b).
Item 9. A neoantigen peptide, comprising an amino acid sequence selected from the group consisting of any one of SEQ ID NO: 1 to SEQ ID NO: 5, and SEQ ID NO: 34, wherein the peptide has the ability to bind to an MHC class I molecule and/or wherein said peptide, when bound to said MHC, is capable of being recognized by CD8 T cells.
Item 10. The neoantigen peptide according to Item 9, selected from the group consisting of SEQ ID NO: 4, and SEQ ID NO: 5, preferably a peptide selected from the group consisting of SEQ ID NO: 4, 5, 7, 8, 9, 15, 17, 29, 31, 32, 33, 37, 39, 44, and 45.
Item 11. The neoantigen peptide according to Item 9 or 10, wherein said peptide has an overall length of from 8 to 30 amino acids.
Item 12. The neoantigen peptide according to any one of Items 9 to 11, wherein said peptide is fused to a different peptide or protein.
Item 13. A neoantigen binding protein that specifically binds to a neoantigen peptide comprising or consisting of an amino acid sequence according to any one of SEQ ID NO: 1 to SEQ ID NO: 5 and SEQ ID NO: 34, preferably a peptide selected from the group consisting of SEQ ID NO: 4, 5, 7, 8, 9, 15, 17, 29, 31, 32, 33, 37, 39, 44, and 45, when said antigenic peptide is in a complex with a major histocompatibility complex (MHC) protein.
Item 14. The neoantigen binding protein according to Item 13, wherein the antigen binding protein is an antibody or a binding fragment thereof, a bispecific, a trispecific, such as a trispecific bi-paratopic, multispecific antibody or multivalent antibody or binding fragment thereof, a chimeric antibody, a humanized antibody, a human antibody, a monoclonal antibody, a deimmunized antibody, a single-chain bispecific antibody, a bi-or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain/nanobody^{®}, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).
Item 15. The neoantigen binding protein according to Item 13 or 14, wherein the antigen binding protein is at least bispecific and furthermore binds to an antigen selected from the group consisting of CD3, CD16, NKG2D, CD28, NKp44, NKp46, OX40, GITR, CD137, CD27, CD40L, HVEM or human glucocorticoid-induced TNFR-related protein (GITR).
Item 16. A nucleic acid molecule encoding the neoantigen peptide according to any one of Items 9 to 12 or the neoantigen binding protein according to any one of Items 13 to 15 or at least one component thereof.
Item 17. A vector comprising a nucleic acid molecule according to Item 16, such as, for example, an expression vector comprising and expressing the nucleic acid molecule.
Item 18. A recombinant or genetically modified cell comprising and optionally expressing the nucleic acid molecule according to claim 16 or the vector according to Item 17, wherein preferably the cell is a T cell, a hybridoma cell, or an antibody excreting cell.
Item 19. A method for producing the neoantigen peptide according to any one of Items 9 to 12, the neoantigen binding protein according to any one of Items 13 to 15 or the at least one component thereof, comprising a) culturing a host cell according to Item 18 under conditions effective to express the neoantigen peptide or the neoantigen binding protein or component thereof; and b) obtaining the expressed neoantigen peptide or the neoantigen binding protein or component thereof from said host cell or the culture medium of said host cell.
Item 20. A method for producing a pharmaceutical composition comprising formulating the neoantigen peptide according to any one of Items 9 to 13, the neoantigen binding protein according to any one of Items 13 to 15 or the at least one component thereof or the recombinant or genetically modified host cell according to Item 18 with a suitable pharmaceutically acceptable carrier, or a pharmaceutical composition as produced.
Item 21. A pharmaceutical composition, produced according to Item 20, preferably a vaccine comprising at least one neoepitope peptide or protein according to any one of SEQ ID NO: 1 to 5, preferably a peptide selected from the group consisting of SEQ ID NO: 4, 5, 7, 8, 9, 15, 17, 29, 31, 32, 33, 37, 39, 44, and 45, or an RNA or DNA molecule encoding the neoepitope peptide or protein.
Item 22. The pharmaceutical composition or vaccine according to Item 21 wherein the neoepitope peptide has been identified by a method according to any one of Items 1 to 8.
Item 23. The vaccine according to Item 21 or 22, comprising at least 2, preferably 3, 4, 5, 6, 7, 8, 9 or 10 or more antigenic peptides comprising at least one neoepitope peptide or protein according to any one of SEQ ID NOs: 1 to 5 and SEQ ID NO: 34, and optionally at least one of a neoepitope peptide or protein according to any one of SEQ ID NOs: 6 to 33, and 35 to 45, more preferably further selected from the group consisting of SEQ ID NO: 7, 8, 9, 15, 17, 29, 31, 32, 33, 37, 39, 44, and 45.
Item 24. The vaccine according to any one of Items 21 to 23, which is in the form of a dendritic cell vaccine, wherein the dendritic cell is pulsed or loaded with the at least one neoepitope peptide or genetically modified to express at least one neoepitope peptide or neoepitope protein.
Item 25. The neoantigen peptide according to any one of Items 9 to 12, the neoantigen binding protein according to any one of Items 13 to 15, the nucleic acid molecule according to Item 16, the vector according to Item 17, the recombinant or genetically modified cell according to Item 18, or the pharmaceutical composition or vaccine according to any one of Items 21 to 23 for use in medicine, or for use in the manufacture of a medicine.
Item 24. The neoantigen peptide according to any one of Items 9 to 12, the neoantigen binding protein according to any one of Items 13 to 15, the nucleic acid molecule according to Item 16, the vector according to Item 17, the recombinant or genetically modified cell according to Item 18, or the pharmaceutical composition or vaccine according to any one of Items 21 to 23 for use in the prevention or treatment for use the treatment of cancer, wherein the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.
Item 27. A method of treating a patient against cancer, the method comprising administering to the patient an effective amount of the neoantigen peptide according to any one of Items 9 to 12, the neoantigen binding protein according to any one of Items 13 to 15, the nucleic acid molecule according to Item 16, the vector according to Item 17, the recombinant or genetically modified cell according to Item 18, or the pharmaceutical composition or vaccine according to any one of Items 21 to 23.
Item 28. The method according to Item 27, wherein the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.
Item 29. A kit comprising a) a container comprising a pharmaceutical composition or vaccine according to any one of Items 21 to 23 in solution or in lyophilized form; and b) optionally, a second container containing a diluent or reconstituting solution for the lyophilized formulation; and c) optionally, at least one more peptide selected from the group consisting of SEQ ID NO: 6 to 33, and 35 to 45, more preferably further selected from the group consisting of SEQ ID NO: 7, 8, 9, 15, 17, 29, 31, 32, 33, 37, 39, 44, and 45.
Item 30. The kit according to Item 28, further comprising d) a composition comprising at least one second pharmaceutically active ingredient, wherein preferably the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine, wherein preferably the provided compositions are to be administered simultaneously or sequentially.

The invention will now be described further in the following examples with reference to the accompanying figures and the attached sequence listing, nevertheless, without being limited thereto. For the purposes of the present invention, all references as cited are incorporated by reference in their entireties.

Figure 1 shows the workflow and characteristics of co-transfection immunopeptidome experiments. (A) Experimental procedure for the identification of HLA ligands from selected antigens using a co-transfection strategy. (B) Relative abundance of KRAS G12V-derived public neoantigen presented by HLA-A*11:01 in various cell numbers (C) Heatmap showing the fraction of presented peptides versus predicted HLA ligands for each protein and HLA allele. Predictions were performed by netMHC4.1. (D-F) Differences of predicted and detected HLA ligands for public neoepitopes regarding predicted HLA binding by (D) eluted ligand rank, (E) binding affinity rank (F) by predicted absolute binding affinity in nM. Unpaired Student's t-test was performed. (G-I) Differences of predicted and detected HLA ligands for wild-type (WT) peptides regarding predicted HLA binding by (G) eluted ligand rank, (H) binding affinity rank, and (I) predicted absolute binding affinity in nM. An unpaired Student's t-test was performed. NS: not significant, *P < 0.05, ****P < 0.000

Figure 2 shows the analysis of HLA/neoantigen combinations, HLA-LOH and clinical T cell responses. (A-B) Heatmap showing screened public neoantigen-HLA combinations. Brown squares indicate undetected neoantigen-HLA pairs. Yellow squares represent detected neoantigen-HLA pairs. Blue squares denote neoantigens that were previously detected from the literature. Squares with centered dots indicate reports of significant HLA loss. Squares with polygon indicate positive clinical T cell responses in patients who received personalized neoantigen based mRNA cancer vaccine.

SEQ ID NOs: 1 to 45 show the neoantigen peptides according to the present invention.

SEQ ID NO: 46 shows a wildtype sequence of TP53 (see SEQ ID NOs: 3 and 30).

**Table 1: Peptides and sequences detected by immunopeptidomics according to the present invention. Peptides of different lengths, derived from various genomically altered driver genes and passenger mutations, are presented across different HLA alleles.**

| HLA Allele | Gene | Mutation | Peptide Sequence | Length | SEQ ID NO: |
|---|---|---|---|---|---|
| HLA-A*02:01 | NOTCH1 | V1575L | RLAAGTLLVVV | 11 | 1 |
| HLA-B*15:01 | MUTYH | Y165C | QLWAGLGCY | 9 | 2 |
| HLA-A*02:01 | TP53 | H193Y | GLAPPQYLIRV | 11 | 3 |
| HLA-A*02:01 | TP53 | P190L | ALPQHLIRV | 9 | 4 |
| HLA-A*33:03 | TP53 | R282W | DWRTEEENLR | 10 | 5 |
| HLA-A*31:01 | AKT1 | E17K | KYIKTWRPR | 9 | 6 |
| HLA-C*07:02 | CTNNB1 | S37F | QSYLDSGIHF | 10 | 7 |
| HLA-A*24:02 | CTNNB1 | S37F | YLDSGIHF | 8 | 8 |
| HLA-A*24:02 | CTNNB1 | S37F | SYLDSGIHF | 9 | 9 |
| HLA-A*03:01 | CTNNB1 | S45F | TTAPFLSGK | 9 | 10 |
| HLA-A*03:01 | CTNNB1 | S45F | TTTAPFLSGK | 10 | 11 |
| HLA-A*03:01 | CTNNB1 | S45F | ATTTAPFLSGK | 11 | 12 |
| HLA-A*03:01 | CTNNB1 | T41A | ATAPSLSGK | 9 | 13 |
| HLA-C*03:04 | EGFR | T790M | STVQLIMQL | 9 | 14 |
| HLA-A*33:03 | EGFR | L858R | HVKITDFGR | 9 | 15 |
| HLA-C* 12:03 | EGFR | T790M | STVQLIMQL | 9 | 16 |
| HLA-A*31:01 | EGFR | L858R | HVKITDFGR | 9 | 17 |
| HLA-A*24:02 | ESR1 | D538G | LYGLLLEML | 9 | 18 |
| HLA-A*11:01 | KRAS | G12A | VVVGAAGVGK | 10 | 19 |
| HLA-A*11:01 | KRAS | G12S | VVVGASGVGK | 10 | 20 |
| HLA-A*11:01 | KRAS | G12V | VVGAVGVGK | 9 | 21 |
| HLA-A*11:01 | KRAS | G12V | VVVGAVGVGK | 10 | 22 |
| HLA-A*03:01 | KRAS | G12R | VVVGARGVGK | 10 | 23 |
| HLA-C*08:02 | KRAS | G12D | GADGVGKSAL | 10 | 24 |
| HLA-A*11:01 | KRAS | G12C | VVVGACGVGK | 10 | 25 |
| HLA-A*11:01 | KRAS | G12D | VVGADGVGK | 9 | 26 |
| HLA-A*11:01 | KRAS | G12D | VVVGADGVGK | 10 | 27 |
| HLA-A*33:01 | PIK3CA | H1047R | DARHGGWTTK | 10 | 28 |
| HLA-B*08:01 | PIK3CA | H1047L | FMKQMNDAL | 9 | 29 |
| HLA-A*02:01 | TP53 | P190L | GLALPQHLIRV | 11 | 30 |
| HLA-A*02:01 | TP53 | P250L | RLILTIITL | 9 | 31 |
| HLA-A*24:02 | TP53 | S241F | HYNYMCNSF | 9 | 32 |
| HLA-A*02:01 | TP53 | R213L | YLDDRNTFL | 9 | 33 |
| HLA-A*03:01 | AKR1A1 | D63N | ALKENVGPGK | 10 | 34 |
| HLA-A*01:01 | CDO1 | E41K | KSDPTEWAMY | 10 | 35 |
| HLA-A*01:01 | PRSS23 | E300D | DVKDDTYDLLY | 11 | 36 |
| HLA-A*02:01 | RADIL | T550M | LMASEEAMAVL | 11 | 37 |
| HLA-A*01:01 | SDK1 | D644N | QTWSGDIGNY | 10 | 38 |
| HLA-A*03:01 | SYT7 | T133M | RMVQSHLAAGK | 11 | 39 |
| HLA-A*03:01 | SYT8 | T133M | MVQSHLAAGK | 10 | 40 |
| HLA-B*07:02 | TAS2R43 | L5R | RPIIFSSL | 8 | 41 |
| HLA-A*02:01 | TP53 | C135Y | YQLAKTCPV | 9 | 42 |
| HLA-A*01:01 | TP53 | I232F | SDCTTFHY | 8 | 43 |
| HLA-B*08:01 | UBE2G2 | E12A | TALKRLMAA | 9 | 44 |
| HLA-B*08:01 | UBE2G2 | E12A | ALKRLMAA | 8 | 45 |

### Examples

### Methods

### Cell lines

The monkey fibroblast COS-7 cell line was acquired through a material transfer agreement (MTA) with the Leibniz Institute DSMZ German Collection of Microorganisms and Cell Cultures GmbH (DSMZ accession number ACC 60). These cells were cultured and maintained in RPMI 1640 medium (Gibco) supplemented with penicillin-streptomycin (Pen-Strep, Gibco) and 10% fetal bovine serum (FBS, GeminiBio).

### Plasmids

Gene sequences for driver genes and HLA alleles utilized in the inventor's research were synthesized and inserted into the pcDNA3.1+ vector by GenScript. The inventors generated constructs encoding full-length driver genes (except for CTNBB1, which could not be produced in full in the inventor's vector) and the HLA alleles of interest. To prepare the RNA for in vitro transcription, the inventors first linearized the plasmid vectors with the Xba1 restriction enzyme (New England Biolabs). The linearized plasmids were then purified using the QIAquick PCR Purification Kit (Qiagen) as per the manufacturer's guidelines. In the next step, the inventors synthesized the in vitro RNA from the purified linearized plasmids using the HiScribe T7 High Yield RNA Synthesis Kit (New England Biolabs), following the protocol provided by the manufacturer.

### Immunopurification of HLA class I ligands

COS-7 cells were co-electroporated with 100 µg/ml of mRNA encoding an individual HLA allele along with the driver protein using the Neon Transfection system (10 µl Ep, 1050 V/30 ms/2 pulses). Approximately 10 × 10⁶ cells were electroporated per condition and subsequently plated in six-well non-tissue culture-treated plates for overnight incubation. The cells were then harvested by incubation with 1 mM EDTA (Millipore Sigma) at 37°C for 5 minutes, followed by pelleting and a single wash in ice-cold RPMI medium (Gibco) and three washes in ice-cold PBS (Gibco). The cells were lysed in 8 ml of 1% CHAPS (Millipore Sigma) for 1 hour at 4°C, after which the lysates were centrifuged at 20,000g for 1 hour at 4°C, and the supernatant was collected. For the immune-purification of HLA-I ligands, 0.5 mg of W6/32 antibody (Bio X Cell) was conjugated to 40 mg of CN Br-activated sepharose (Cytiva) and incubated with the protein lysate overnight. HLA complexes along with binding peptides were then eluted five times using 1% TFA. These peptides and HLA-I complexes were separated using C18 columns or C18 column material (Sep-Pak C18 1 cc Vac Cartridge, 50 mg sorbent, 37-55 µm particle size, Waters), which were preconditioned with 80% Acetonitrile (ACN) (Millipore Sigma) and equilibrated with three washes of 0.1% TFA. Samples were loaded onto the columns twice, washed twice with 0.1% TFA, and then eluted in 200 µl of sequentially increased ACN concentrations - 15%, 30%, 40%, and 50% in 0.1% TFA. The four fractions were combined, dried using vacuum centrifugation, and stored at -80°C until further processing.

### Solid-phase extractions

In-house C18 minicolumns were prepared using the following method: for the solid-phase extraction of a single sample, two small disks (1 mm diameter each) of C18 material were punched out from CDS Empore C18 disks (Thermo Fisher Scientific).

These disks were then placed at the bottom of a 200 µl Axygen pipette tip (Thermo Fisher Scientific). The columns were initially washed with 100 µL of 80% ACN in 0.1% TFA and subsequently equilibrated three times with 100 µl of 1% TFA. Fluids were passed through the column by centrifugation in a mini tabletop centrifuge, and the eluates were collected in Eppendorf tubes. Dried samples were then resuspended in 100 µl of 1% TFA, loaded onto the columns, washed twice with 100 µl of 1% TFA, and then run dry. Finally, the samples were eluted with 60 µl of 60% ACN/0.1% TFA, and the resulting sample volume was further reduced by vacuum centrifugation.

### Liquid chromatography-tandem mass spectrometry analysis of HLA ligands

Samples were analyzed using high-resolution and high-accuracy liquid chromatography-tandem mass spectrometry (LC-MS/MS) on a Lumos Fusion system (Thermo Fisher Scientific). Peptide separation was achieved by direct loading onto a packed-in-emiper C18 column (75 µm inner diameter, 12 cm length, 3 µm particles, Nikkyo Technos Co., Ltd.). The gradient was applied at a flow rate of 300 nl/min, starting with 2% buffer B (0.1% formic acid in 80% acetonitrile) and 98% buffer A (0.1% formic acid), and linearly increasing to 30% buffer B and 70% buffer A over 70 minutes. Mass spectrometry (MS) and tandem mass spectrometry (MS/MS) analyses were conducted with resolutions set to 60,000 and 30,000, respectively. Only peptide ions with charge states of +1, +2, and +3 were selected for analysis. The isolation window was set to 1.6 m/z, and the collision energy was fixed at 30%. For MS/MS, the maximum injection time was 100 milliseconds, with an automatic gain control target of 50,000.

### MS data processing

Mass spectrometry data were processed using Byonic software (version 2.7.84, Protein Metrics) and Peaks software (version 11, Bioinformatics Solutions Inc.) on a custom-built server equipped with 4 Intel Xeon E5-4620 8-core CPUs at 2.2 GHz and 512 GB of RAM (Exxact Corporation). The mass accuracy was set to 6 ppm for MS1 and 20 ppm for MS2. Digestion was specified as unspecific, allowing only precursors with charges of +1, +2, and +3 and a maximum mass of 2 kDa. Protein FDR was turned off to allow complete assessment of potential peptide identifications. Variable modifications included methionine oxidation; phosphorylation of serine, threonine, and tyrosine; and N-terminal acetylation. Samples were analyzed against a database containing UniProt Cercopithecus aethiops reviewed proteins, supplemented with human mutant sequences of driver genes used in the study and common contaminants. Peptides ranging from 8 to 15 amino acids in length were selected with a minimum log probability value of 1.3, corresponding to P-values of 0.05, and duplicates were removed. Ion intensities were exported from Byonic and Peaks for mirror plots. For peak area and retention time analysis, Skyline software (version 24.1, MacCoss Lab Software) was utilized. Precursor masses of target peptide sequences were searched in all relevant raw files, and peak areas were compared. Retention Emes for the best-scoring matches were determined using a total of four isotopes.

### Assignment of peptide sequences to HLA alleles

To assign peptides that passed the MS quality filters to their most likely HLA complexes, the inventors employed the netMHCpan 4.1 algorithm (DTU Health Tech) with its default settings. Peptides with an affinity percentage rank below 2 were classified as binders.

### Flow cytometry analysis

For the surface HLA staining procedure, approximately 2X10⁵ cells in 200 µl of medium were transferred into a 96-well V-bottom plate. The cells were centrifuged at 1400 rpm for 5 minutes and then stained with 50 µl of a primary antibody specific to the surface molecule of interest, diluted in FACS buffer (PBS/3% BSA/0.01% NaN₃), for 15 minutes at 4°C in the dark. Following two washing steps with FACS buffer, the samples were resuspended in FACS buffer and transferred to FACS tubes for analysis using a Beckman Coulter flow cytometer. For intracellular protein staining, approximately 2X105 cells were fixed and permeabilized according to the BD Cytofix/Cytoperm^{™} Fixation/Permeabilization Kit protocol (BD Biosciences). The cells were then stained with 50 µl of a primary antibody specific to the intracellular molecule of interest, diluted in Perm/Wash buffer (BD Biosciences), for 15 minutes at 4°C. Aker two washing steps with Perm/Wash buffer, the cells were incubated with a fluorescently labeled secondary antibody that targets the isotype of the primary antibody for 15 minutes at 4°C in the dark. Finally, the samples were washed again, resuspended in FACS buffer, and transferred to FACS tubes for acquisition using a Beckman Coulter flow cytometer.

### Statistics

All graphs except heatmaps were drawn with GraphPad Prism version 10 software. Appropriate statistical tests were used to analyze data, as described in each figure legend.

Statistical analyses were performed with GraphPad Prism version 10 software. Significance was preset at P < 0.05

### Mutated HLA ligand peptides can preferably be detected by immunopeptidomics according to the present invention when compared to their non-mutated sequences

The capacity of this approach to identify mutated HLA ligands rather than their non-mutated counterparts is supported as follows.

First, if the mutated amino acid is in a non-anchor position of the peptide (e.g., GLAPPQYLIRV (SEQ ID No. 3), GLALPQHLIRV (SEQ ID No. 30)) the present method successfully detected the corresponding mutated peptides but not the non-mutated versions that were actually reported to be presented (https://www.iedb.org/hom_v3.php, the link refers to 31 mass spectrometry identified sequences GLAPPQHLIRV (SEQ ID No. 46) which is the corresponding wildtype sequence. The sequence needs to be entered in the linear peptide section and go to assays and then MHC ligand assays). Moreover, experiments that included TP53 but a mutation in a different section of the protein, e.g, R213L did not only identify the mutated sequence SEQ ID No. 33 = YLDDRNTFL but also the wildtype sequence GLAPPQHLIRV (SEQ ID No. 46).

Second, if the mutated amino acid is in an anchor position of the peptide (e.g., ALPQHLIRV (SEQ ID No. 4), RLILTIITL (SEQ ID No. 31)) the present method successfully detected the mutated sequences and did not lead to identification of the non-mutated sequences, e.g. APPQHLIRV, RPILTIITL although it must be noted that the sequence RPILTIITL is presented in the context of HLA-B*07:02 (https://www.iedb.org/home_v3.php, and then enter RPILTIITL in the linear peptide field as mentioned above). These wildtype sequences are not detected when using the present method other TP53 mutations in the context of HLA-A*02:01 which underlines the specificity of the present method to identify mutated sequences but not wildtype sequences if these are not presented in the context of the respective HLA allele.

**Table 2: Preferred mutated HLA ligand peptides that were preferably detected by immunopeptidomics according to the present invention compared to their non-mutated sequences. As a wildtype, all these peptides sequence can not bind to the corresponding HLA-type.**

| HLA Allele | Gene | Mutation | Peptide Sequence | Length | SEQ ID NO: |
|---|---|---|---|---|---|
| HLA-A*02:01 | TP53 | P190L | ALPQHLIRV | 9 | 4 |
| HLA-A*33:03 | TP53 | R282W | DWRTEEENLR | 10 | 5 |
| HLA-C*07:02 | CTNNB1 | S37F | QSYLDSGIHF | 10 | 7 |
| HLA-A*24:02 | CTNNB1 | S37F | YLDSGIHF | 8 | 8 |
| HLA-A*24:02 | CTNNB1 | S37F | SYLDSGIHF | 9 | 9 |
| HLA-A*33:03 | EGFR | L858R | HVKITDFGR | 9 | 15 |
| HLA-A*31:01 | EGFR | L858R | HVKITDFGR | 9 | 17 |
| HLA-B*08:01 | PIK3CA | H1047L | FMKQMNDAL | 9 | 29 |
| HLA-A*02:01 | TP53 | P250L | RLILTIITL | 9 | 31 |
| HLA-A*24:02 | TP53 | S241F | HYNYMCNSF | 9 | 32 |
| HLA-A*02:01 | TP53 | R213L | YLDDRNTFL | 9 | 33 |
| HLA-A*02:01 | RADIL | T550M | LMASEEAMAVL | 11 | 37 |
| HLA-A*03:01 | SYT7 | T133M | RMVQSHLAAGK | 11 | 39 |
| HLA-B*08:01 | UBE2G2 | E12A | TALKRLMAA | 9 | 44 |
| HLA-B*08:01 | UBE2G2 | E12A | ALKRLMAA | 8 | 45 |

### Literature as cited

1. D'Angelo, S. P. et al. The Lancet 403, 1460-1471 (2024).
2. Nathan, P. et al. N Engl J Med 385, 1196-1206 (2021).
3. Rojas, L. A. et al. Nature 618, 144-150 (2023).
4. Saotome, K. et al. Nat Commun 14, 2401 (2023).
5. Solanki, A., Riedel, M., Cornette, J., Udell, J. & Vasmatzis, G. Front. Oncol. 12, 1034810 (2022).
6. Chowell, D. et al. Nat Med 25, 1715-1720 (2019).
7. Montesion, M. et al. Cancer Discovery 11, 282-292 (2021).
8. Wang, Q. et al. Cancer Immunology Research 7, 1748-1754 (2019).
9. Chandran, S. S. et al. Nat Med 28, 946-957 (2022).
10. Klatt, M. G. et al. Journal of Proteomics 228, 103921 (2020).
11. Klatt, M. G. et al. 5, e141264 (2020).
12. Lo, W. et al. Cancer Immunology Research 7, 534-543 (2019).
13. Hsiue, E. H.-C. et al. Science 371, eabc8697 (2021).
14. Gurung, H. R. et al. Nat Biotechnol 42, 1107-1117 (2024).
15. Choi, J. et al. Cell Rep Methods 1, 100084 (2021).
16. Ott, P. A. et al. Nature 547, 217-221 (2017).
17. Sahin, U. et al. Nature 547, 222-226 (2017).
18. Luksza, M. et al. Nature 551, 517-520 (2017).
19. Wells, D. K. et al. Cell 183, 818-834.e13 (2020).
20. Immisch, L. et al. J Immunother Cancer 10, e005535 (2022).

## Claims

1. An improved method for identifying at least one HLA ligand neoantigen peptide derived from at least one mutated antigen protein, comprising the steps of
a) co-transfecting a suitable mammalian cell with mRNA from the at least one mutated antigen and mRNA from at least one preselected HLA allele,
b) culturing the cell to express HLA/neoantigen peptide complexes,
c) isolating the HLA/neoantigen peptide complexes from the culture,
d) separating the neoantigen peptide from the HLA proteins using concentrations of between 40 and 50% of acetonitrile,
e) analyzing the at least one neoantigen peptide comprising mass spectrometry (MS) analysis, and
f) identifying the least one neoantigen peptide.

2. The method according to claim 1, wherein the cell that expresses the HLA/neoantigen peptide complexes is selected from the group of COS-7, AML14, JMN, MCF-7, and BV173 cells.

3. The method according to claim 1 or 2, wherein the isolating comprises immunoprecipitation, in particular HLA immunoprecipitation, and/or wherein the isolating comprises the use of C18 HPLC column material.

4. The method according to any one of claims 1 to 3, wherein the amount of HLA/neoantigen peptide complex as isolated is higher than about 5 fmol per neoantigen peptide.

5. The method according to any one of claims 1 to 4, wherein the at least one neoantigen peptide as identified comprises a mutation compared to the wildtype sequence of the antigen, and wherein non-mutated peptides of the antigen are not presented in the context of the respective HLA allele.

6. A neoantigen peptide, comprising an amino acid sequence selected from the group consisting of any one of SEQ ID NO: 1 to SEQ ID NO: 5, and SEQ ID NO: 34, wherein the peptide has the ability to bind to an MHC class I molecule and/or wherein said peptide, when bound to said MHC, is capable of being recognized by CD8 T cells, preferably selected from the group consisting of SEQ ID NO: 3, and SEQ ID NO: 4, preferably further selected from the group consisting of SEQ ID NO: 7, 8, 9, 15, 17, 29, 31, 32, 33, 37, 39, 44, and 45.

7. A neoantigen binding protein that specifically binds to a neoantigen peptide comprising or consisting of an amino acid sequence according to any one of SEQ ID NO: 1 to SEQ ID NO: 5 and SEQ ID NO: 34, preferably selected from the group consisting of SEQ ID NO: 3, and SEQ ID NO: 4, preferably further selected from the group consisting of SEQ ID NO: 7, 8, 9, 15, 17, 29, 31, 32, 33, 37, 39, 44, and 45, when said antigenic peptide is in a complex with a major histocompatibility complex (MHC) protein, wherein preferably the antigen binding protein is an antibody or a binding fragment thereof, a bispecific, a trispecific, such as a trispecific bi-paratopic, multispecific antibody or multivalent antibody or binding fragment thereof, a chimeric antibody, a humanized antibody, a human antibody, a monoclonal antibody, a deimmunized antibody, a single-chain bispecific antibody, a bi- or tri-specific T-cell engager (BiTE), a TCR mimic (TCRm) antibody, a multi-specific antibody formed from antibody fragments, or a combination thereof, or wherein the antigen binding protein or a binding fragment thereof comprises a Fab, Fab', Fab'-SH, Fv, scFv, F(ab')₂, a diabody, a linear antibody, a single domain antibody (sdAb), a camelid VHH domain/nanobody^{®}, or wherein the antigen binding protein is a T cell receptor (TCR), such as an alpha/beta or gamma/delta receptor, or a binding fragment thereof, a bispecific T cell receptor (TCR) or binding fragment thereof, a single chain TCR (scTCR) or a chimeric antigen receptor (CAR).

8. The neoantigen binding protein according to claim 7, wherein the antigen binding protein is at least bispecific and furthermore binds to an antigen selected from the group consisting of CD3, CD16, NKG2D, CD28, NKp44, NKp46, OX40, GITR, CD137, CD27, CD40L, HVEM or human glucocorticoid-induced TNFR-related protein (GITR).

9. A nucleic acid molecule encoding the neoantigen peptide according to claim 6 or the neoantigen binding protein according to claim 7 or 8 or at least one component thereof, or a vector comprising said nucleic acid molecule, such as, for example, an expression vector comprising and expressing the nucleic acid molecule.

10. A recombinant or genetically modified cell comprising and optionally expressing the nucleic acid molecule or the vector according to claim 9, wherein preferably the cell is a T cell, a hybridoma cell, or an antibody excreting cell.

11. A method for producing a pharmaceutical composition comprising formulating the neoantigen peptide according to claim 6, the neoantigen binding protein according to claim 7 or 8 or the at least one component thereof or the recombinant or genetically modified host cell according to claim 10 with a suitable pharmaceutically acceptable carrier, or a pharmaceutical composition as produced.

12. A pharmaceutical composition, produced according to claim 11, preferably a vaccine comprising at least one neoepitope peptide or protein according to any one of SEQ ID NO: 1 to 5, or an RNA or DNA molecule encoding the neoepitope peptide or protein, wherein preferably the neoepitope peptide has been identified by a method according to any one of claims 1 to 5.

13. The neoantigen peptide according to claim 6, the neoantigen binding protein according to claim 7 or 8, the nucleic acid molecule or vector according to claim 9, the recombinant or genetically modified cell according to claim 10, or the pharmaceutical composition or vaccine according to claim 12 for use in medicine, or for use in the manufacture of a medicine, preferably for use in the prevention or treatment for use the treatment of cancer, wherein the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.

14. A method of treating a patient against cancer, the method comprising administering to the patient an effective amount of the neoantigen peptide according to claim 6, the neoantigen binding protein according to claim 7 or 8, the nucleic acid molecule or vector according to claim 9, the recombinant or genetically modified cell according to claim 10, or the pharmaceutical composition or vaccine according to claim 12, wherein preferably the cancer is selected from the group consisting of acute myeloid leukemia, breast cancer, cholangiocellular carcinoma, chronic lymphocytic leukemia, colorectal cancer, gallbladder cancer, glioblastoma, gastric cancer, gastro-esophageal junction cancer, hepatocellular carcinoma, head and neck squamous cell carcinoma, melanoma, non-Hodgkin lymphoma, non-small cell lung cancer, ovarian cancer, esophageal cancer, pancreatic cancer, prostate cancer, renal cell carcinoma, small cell lung cancer, urinary bladder carcinoma, and uterine endometrial cancer.

15. A kit comprising a) a container comprising a pharmaceutical composition or vaccine according claim 12 in solution or in lyophilized form; and b) optionally, a second container containing a diluent or reconstituting solution for the lyophilized formulation; and c) optionally, at least one more peptide selected from the group consisting of SEQ ID NO: 6 to 33, and 35 to 45, and optionally further comprising d) a composition comprising at least one second pharmaceutically active ingredient, wherein preferably the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine, wherein preferably the provided compositions are to be administered simultaneously or sequentially.
